# EUROPEAN PATENT APPLICATION

(11) **EP 4 759 242 A1**
(43) Date of publication of application: **17.06.2026**
(21) Application number: 24218934.8
(22) Date of filing: 11.12.2024
(51) Int. Cl.: A61B 6/40, A61B 6/50, A61B 6/51, A61B 6/00

(54) **COMPUTER-IMPLEMENTED METHOD FOR X-RAY IMAGING WITH VARYING X-RAY DOSE**

(71) Applicant: Dentsply Sirona Inc., York, PA 17401 (US); SIRONA Dental Systems GmbH, 64625 Bensheim (DE)
(72) Inventor: Elvers, Michael, 64625 Bensheim (DE); Ulrici, Johannes, 64625 Bensheim (DE)
(74) Representative: Aldridge, Henry Alexander

(57) **Abstract**

The present invention relates to a computer-implemented method for generating a two dimensional final image (16) of at least a sub region of the maxillofacial region of a patient (P) with x-ray cone beam CT, the method comprising the steps of: defining a position and alignment of the 2D final image to be generated relative to said sub region of the dental maxillofacial region of the patient, wherein the 2D final image essentially being identical to a x-ray projection image of said sub region with parallel beam geometry; acquiring a plurality of x-ray projection images by rotating an x-ray source (3) and an x-ray detector (4) around the head of the patient, wherein the x-ray source and the x-ray detector are configured for a x-ray cone beam CT image acquisition; varying, during the acquisition step, the amounts of x-ray doses used for the acquisition of each of the x-ray projection images such that a total amount of the x-ray dose is increased above a predetermined level for the x-ray projection images that are acquired when the projection direction is within a first predetermined range of angles (a) centered about a first direction which is parallel to the normal direction of the 2D final image to be generated, and a total amount of the x-ray dose is reduced below the predetermined level for the x-ray projection images that are acquired when the projection direction is within a second predetermined range of angles (b) centered about a second direction which is perpendicular to the normal direction of the 2D final image to be generated; generating the 2D final image as a virtual projection with parallel x-ray beam geometry of the sub region of the maxillofacial region of a patient by using the x-ray projection images acquired.

## Description

### TECHNICAL FIELD OF THE INVENTION

The present invention relates to methods and systems for x-ray imaging of patients in the dental field.

### BACKGROUND ART OF THE INVENTION

Volume images, tomosynthesis based images such as panoramic images, and ceph images are among the standard imaging procedures of all extraoral dental radiographic examinations. Volume images, tomosynthesis based images, and ceph images provide the dentist with information about the patient's dental condition. These images are used by dentists to diagnose problem cases and prepare for treatment.

A cephalometric (ceph) image (cephalogram) is part of an orthodontist's standard diagnostics. Ceph images are required, among other things, to determine relevant positions in the patient's jaw for the planning, implementation, and follow-up of an orthodontic treatment.

Various digital ceph systems exist in today's dental market for extraoral x-ray systems.

On the one hand, there is the so-called one-shot ceph, whereby a correspondingly large, expensive digital ceph sensor is used to capture the patient's head directly with only one short static exposure. This prevents motion artefacts, for example, due to the reduced exposure time, but such ceph sensors are often expensive and bulky. Such a one-shot ceph system is not part of this application.

On the other hand, linear scan systems are another option for creating a ceph image. Here, the patient is sequentially scanned at the required focus-detector distance (usually approx. >1.5 m). In addition to a primary aperture in the x-ray source, an additional secondary aperture associated with the ceph detector is commonly used for additional x-ray dose reduction. The exposure time is longer than with one-shot ceph, but the ceph sensor costs (i.e., a digital ceph sensor based on CCD/CMOS) are significantly lower. Due to the sequential recording technology, however, the demands on the x-ray device (mechanics, motors) are significantly higher, as the ceph sensor used must be moved synchronously with the secondary aperture along the patient's head during the recording.

Older linear scan systems based on this approach use CCD-based ceph sensors according to the commonly known TDI principle (i.e., time delay integration). This means that the summation of the image information takes place directly in the ceph sensor and one image column of the ceph image to be created is always output sequentially by such a ceph sensor. The integration time and readout speed are determined by the TDI frequency. However, a subsequent analysis of the image information per acquisition time through software is not possible with this TDI-based linear scan system variant. With the TDI method, all individual image information is generally processed directly in the ceph sensor.

In general, mechanical and motor-related deviations from the target behavior of the linear scan system during operation can lead to disturbances in the acquisition process, which in turn cause image artifacts, including so-called chatter marks in the ceph image. These reduce the image quality and diagnostic suitability (i.e., risk of misdiagnosis) of the ceph images and may lead to repeat exposure. When the secondary aperture and the ceph detector are not synchronously aligned during the linear scan (e.g., when the center of the secondary aperture is not synchronously coincided with the center of the exposed frame being read out), the grey level peak of the distributions of the image data (i.e., generally a gaussian-like distribution across the secondary aperture) captured by the ceph sensor are relatively shifted from their ideal centered positions on the ceph detector, which in turn leads during the add & shift reconstruction of the ceph image to grey-level intensity variations which are conspicuous in form of vertical and/or horizontal stripes. Thus, the frame being read out must be uniformly exposed. Examples of chatter marks can be seen in the ceph image as non-patient-specific fluctuations in the intensity of the gray values. Especially in a so-called technical ceph images (i.e., a ceph image without a patient), chatter marks can also be seen as shown due to clear fluctuations in the intensity of the gray values.

The above-mentioned one-shot sensors for ceph imaging solve the chatter mark problem completely but are very expensive and not possible on every extraoral x-ray system.

Some extraoral x-ray systems with linear scan ceph technology completely dispense with the secondary aperture to avoid the chatter mark problem, however which can lead to a higher dose and thus to unnecessary patient hazard. With liner-scan ceph technology without a secondary aperture, the scan speed can be increased to keep the dose constant, but this can lead to a reduction in image quality.

Alternatively, ceph images can be generated without using a separate ceph unit, but a dental bone beam CT. Generally, an extraoral x-ray cone beam imaging system has an x-ray source and an x-ray detector that are rotated around the patient's head during the x-ray imaging. The trajectory of the x-ray source and the x-ray detector during the imaging can describe various paths. During the rotation the x-ray detector acquires x-ray projection images of the patient. The panoramic image is generally reconstructed from the x-ray projection images using standard techniques such a shift-and-add or other techniques known to those skilled in the art. A panoramic image shows the structures located in a physical layer to be sharply viewed within the patient's jaw. The Volume image is generally reconstructed from the x-ray projection images using a standard technique such as the filtered back projection algorithm (e.g., the Feldkamp algorithm). The Ceph image can be generated from the volume image.

In standard dental Cone Beam CT, x-ray projection images are acquired from different positions around the object. Spatial resolution of the x-ray sensor is kept constant for the different image positions. A 3D volume is then reconstructed from the projections. SNR on the sensor at a given spatial resolution and a given anatomical object depends on the radiation dose arriving at the detector, i.e. in case of higher spatial resolution, keeping the SNR at the same level will require a higher dose. In case the requirement on spatial resolution for different image acquisition position differs for some projections without effectively reducing spatial resolution, too much dose will be applied to the patient.

### SUMMARY OF THE INVENTION

An objective of the present invention is to provide a computer-implemented method and system for generating a two-dimensional final image of at least a sub region of the maxillofacial region of a patient with x-ray cone beam CT while reducing the overall dose.

These objectives have been achieved by the computer-implemented method as defined in claim 1, and the x-ray imaging system as defined in claim 6. The subject-matters of the dependent claims define further developments and preferred embodiments.

The computer-implemented method of the present invention is suitable for generating a two-dimensional final image of at least a sub region of the maxillofacial region of a patient with x-ray cone beam CT. The method comprises: A step of defining a position and alignment of the 2D final image to be generated relative to said sub region of the dental maxillofacial region of the patient, wherein the 2D final image essentially being identical to a x-ray projection image of said sub region with parallel beam geometry;

a step of acquiring a plurality of x-ray projection images by rotating an x-ray source and an x-ray detector around the head of the patient, wherein the x-ray source and the x-ray detector are configured for a x-ray cone beam CT image acquisition; a step of varying, during the acquisition step, the amounts of x-ray doses used for the acquisition of each of the x-ray projection images such that a total amount of the x-ray dose is increased above a predetermined level for the x-ray projection images that are acquired when the projection direction is within a first predetermined range of angles centered about a first direction which is parallel to the normal direction of the 2D final image to be generated, and such that a total amount of the x-ray dose is reduced below the predetermined level for the x-ray projection images that are acquired when the projection direction is within a second predetermined range of angles centered about a second direction which is perpendicular to the normal direction of the 2D final image to be generated; and a step of generating the 2D final image as a *virtual projection* with parallel x-ray beam geometry of the sub region of the maxillofacial region of a patient by using the x-ray projection images acquired According to a preferred embodiment of the present invention, during the acquiring step the x-ray projection images are read out from the x-ray detector with a higher resolution compared to a predetermined resolution when the x-ray dose is relatively increased; and the x-ray projection images are read out from the x-ray detector with a lower resolution compared to the said predetermined resolution when the x-ray dose is relatively decreases.

According to a preferred embodiment of the present invention, in the generating step, a volume image is reconstructed by using the plurality of x-ray projection images, and then the 2D final image is generated by virtually projecting with parallel x-ray beam geometry the sub region of the volume image.

According to another preferred embodiment of the present invention the 2D final image is a ceph image.

According to a further preferred embodiment of the present invention in the varying step, the speed of revolution and/or the emission of x-ray sources is changed for adaptively varying the amounts of x-ray dose.

The present invention also includes a computer program comprising computer-readable code which, when executed by a computer-assisted x-ray cone beam imaging system, causes the said system to perform the method.

The present invention also includes an extraoral x-ray cone beam imaging system, comprising: an x-ray source and an x-ray detector which are rotatably arranged around a patents' head; a control means for controlling the x-ray source and the x-ray detector; wherein the control means is configured to execute the method. The extraoral x-ray cone beam imaging system can be with a ceph unit or preferably without a dedicated ceph unit.

### BRIEF DESCRIPTION OF THE DRAWINGS

In the following description, the present invention will be explained in more detail with reference to the exemplary embodiments and with reference to the drawings, wherein.
- Fig. 1 -: shows a schematic drawing of an extraoral x-ray cone beam system having a CEPH imaging modality according to an embodiment of the present invention;
- Fig. 2: is a schematic drawing of an x-ray cone beam system with a PAN/DVT imaging modality according to an embodiment of the present invention;
- Fig. 3: is a schematic drawing of x-ray cone beam projection imaging (left side), and a parallel x-ray beam virtual projection imaging (right side) according to an embodiment of the present invention;
- Fig. 4: is a schematic drawing of a sequence of x-ray cone beam projection imaging with a flat-panel x-ray detector having a readout region according to an embodiment of the present invention;

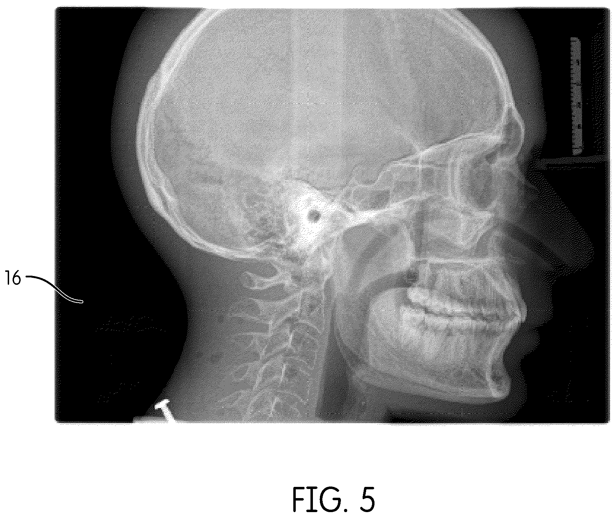

- Fig. 5: shows a ceph image of a phantom head according to the present invention according to an embodiment of the present invention generated according to an embodiment of the present invention;

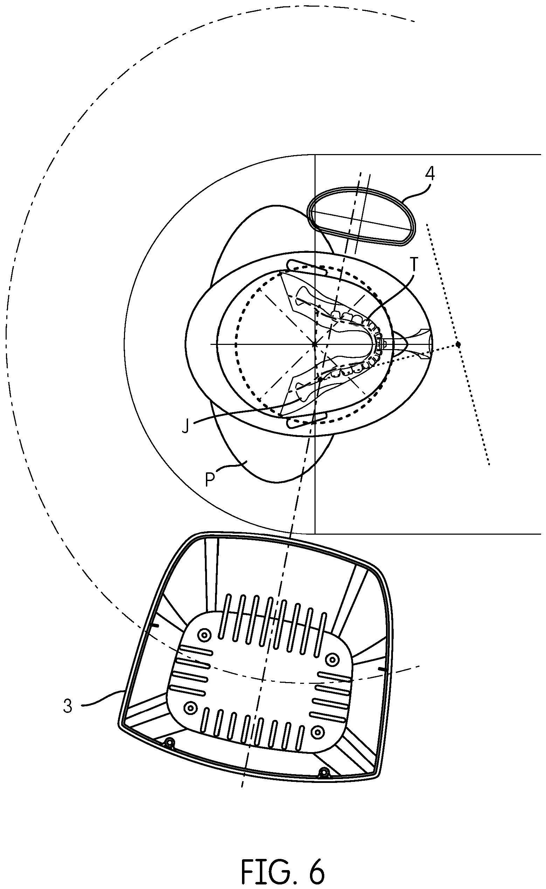

- Fig. 6: is a partial schematic drawing of the x-ray system according to an embodiment of the present invention, during the x-ray cone beam projection imaging when the molar region is exposed;

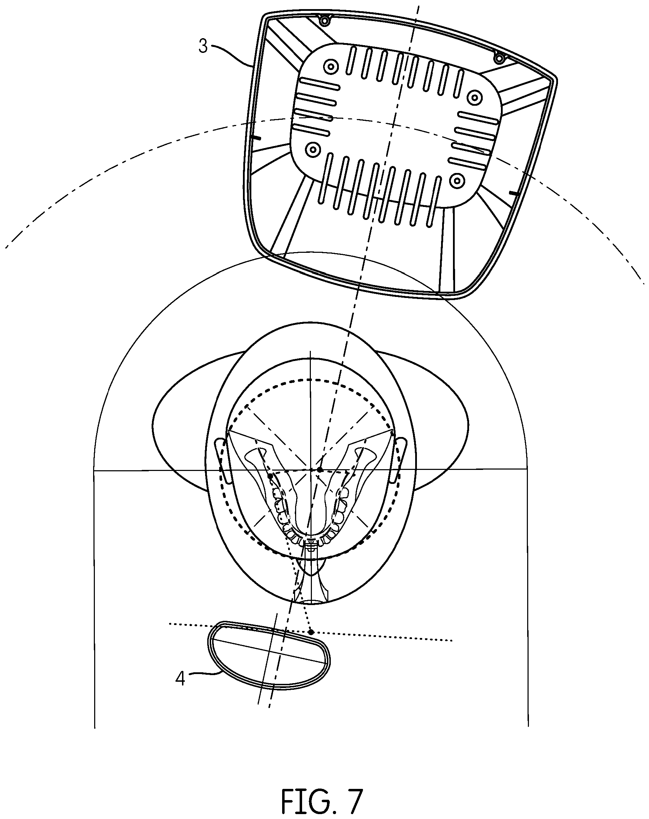

- Fig. 7: is a partial schematic drawing of the x-ray system according to an embodiment of the present invention, during the x-ray cone beam projection imaging when the incisal region is exposed.

The reference numbers shown in the drawings designate the elements listed below, which are referred to in the following description of the exemplary embodiments.
1. Extraoral x-ray system
2. X-ray device
3. X-ray source
4. Primary x-ray detector (PAN/DVT detector)
5. Primary collimator (dashed lines)
6. Cantilever arm
7. Secondary x-ray detector (CEPH detector)
8. Secondary collimator (CEPH collimator)
9. Operating unit (user interface)
10. 10' Head fixation
11. Bite block
12. Computing unit (Computer)
13. Display device (Screen)
14. Input device (Keyboard)
15. Input device (Mouse)
16. 2D final image (Cephalometric image)
17. Read out region
   A: Position of the Patient' head for CEPH imaging
   B: Position of the Patient's head for PAN/DVT imaging
   a,b: Range of angles
   J: Jaw bone
   P: Patient:
   T: Teeth

### DETAILED DESCRIPTION OF THE EMBODIMENTS

In the following description, various x-ray imaging systems (1) and x-ray imaging methods according to the present invention will be explained in more detail.

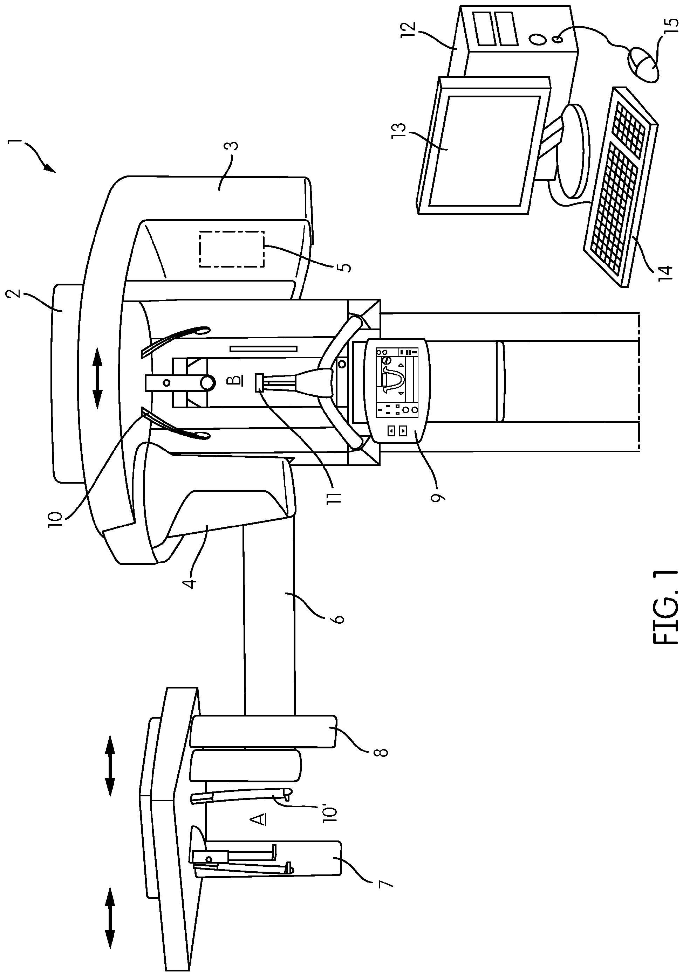

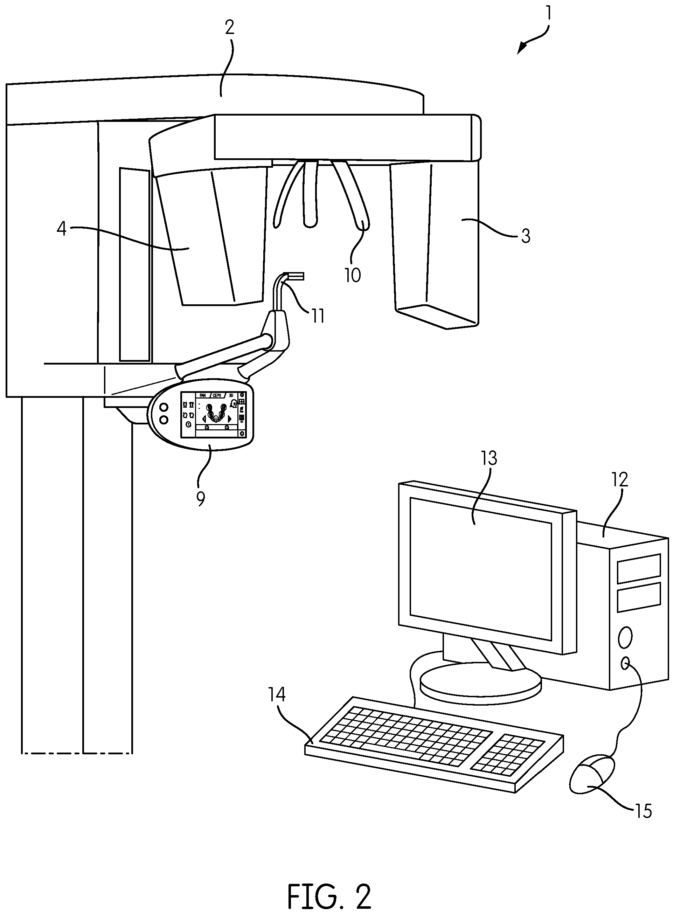

The method according to the present invention, which is explained in more detail below, is a computer-implemented method (i.e., herein also referred to as a software) and can be carried out on a computer-implemented extraoral x-ray system (1) as shown in an embodiment Fig. 2. The computer-implemented method (i.e., herein also referred to as a software) can be also carried out on a computer-implemented extraoral x-ray system (1) as shown in an embodiment in Fig. 1 without using the CEPH unit.

The present invention also includes a corresponding computer program (i.e., the software) having computer-readable code for implementing the method. The computer program is provided on a computer readable storage medium accessible to the extraoral x-ray system (1).

The computerized extraoral x-ray system (1) shown in Fig. 1 is a multifunctional system for panoramic imaging, digital volume tomographic imaging, and cephalometric imaging.

The extraoral x-ray system (1) comprises an x-ray source (3) and a primary x-ray detector (4) that are rotatably arranged around the patient's head for PAN/DVT imaging (see position B in Fig. 1). The x-ray source (3) and the x-ray detector (4) are on a gantry. The x-ray source (3) has a primary collimator (5). The extraoral x-ray system (1) also has a cantilever arm (6) that holds a secondary x-ray detector (7) (i.e., the ceph detector) and a secondary collimator (8) (i.e., the ceph collimator). Preferably a CMOS-based ceph detector is used. This CMOS-based ceph detector is preferably a detector with a scintillator or a direct converting detector. The x-ray ceph detector (7) and the x-ray ceph collimator (8) can be linearly moved along the patient's head (see position A in Fig. 1). A sequence of projection images of the patient's head can be obtained by exposing the patient's head with x-rays from the x-ray source (3) while linearly moving the x-ray ceph detector (7) and the x-ray ceph collimator (8) on opposite sides along the patient's head, while the head being positioned between them. See Position A in Fig. 1 for the patient's head in the ceph mode. In the ceph mode, the primary x-ray detector (4) is automatically moved out of the x-ray beam path to a side position to avoid blocking the x-rays that are emitted from the x-ray source (3) towards the ceph collimator (8). The patient's head can be positioned in the x-ray ceph system (1) with a head fixation (10'). The x-ray ceph collimator (8) has a ceph aperture structure (not shown). The x-ray ceph collimator (8) can be provided as single component aperture structure or as an adjustable (motorized) multicomponent aperture structure, wherein at least the vertically extending aperture blade components can be moved sideways by a motorized mechanism to adjust the width of the aperture. Said components can be made from x-ray opaque/absorbing material.

In the PAN/DVT mode, the trajectory of the x-ray source (3) and the x-ray detector (4) during the PAN/DVT imaging can describe a circular path. However, it can also assume a form deviating from this. If several actuators (not shown) are controlled simultaneously, a trajectory deviating from a pure circular path around the patient's head can be achieved. The patient's head can be positioned with a bite block (11) and optionally with a head fixation (10). See Position B in Fig. 1 for the patient's head in the PAN/DVT mode. The trajectory (*course*) of the x-ray source (3) and the primary x-ray detector (4) with respect to the bite block (11) and the head fixation (10) are known by the system (1). The primary x-ray detector (4) detects the x-rays emitted by the x-ray source (3) during the rotation. The x-ray projection images for PAN/DVT imaging are acquired, namely read out from the x-ray detector (4). The Primary x-ray detector (PAN/DVT detector) has a separate PAN detector and a separate DVT detector which can be rotated around the vertical axis to face the x-ray source (3) in accordance with the PAN/DVT imaging respectively. Or it could be a single flat panel detector capable for PAN as well as DVT.

The computerized extraoral x-ray system (1) also comprises: an operating unit (9) such as a user interface for controlling all functionalities of the modes; a computing unit (12) e.g., a computer; and a display device (13) such as a screen for visualizing any data sets (projections images and the like) resulting from the software. The CEPH/PAN/DVT modes can be each selected by the user e.g., through the operating unit (9) and/or the computer unit (12). The computer may be connected to the extraoral x-ray system (1) via a local area network (not shown) or, alternatively, via the Internet. The computer is connected to input devices such as a keyboard (14), mouse (15), and the like. The computer may be also part of a cloud. Alternatively, the computer may be integrated into the extraoral x-ray system (1). Alternatively, all or some of the computations may take place in the cloud as cloud computing or on an FPGA (field programmable gate array) in hardware. The computer executes the computer program and provides the data sets, e.g., the cephalometric images, the panoramic images, and/or the DVT images for visualization on the screen. The screen may be provided spatially separate from or integrated with the extraoral x-ray system (1). Preferably, the computer may also control all functions of the extraoral x-ray system (1). Alternatively, separate computers may be used for the control, operation, and image reconstruction.

The extraoral x-ray system (1) is preferably configurable as an IoT system and can be bidirectionally connected via a local area network and/or the internet (not shown) to other dental devices (not shown) such as optical intraoral scanners, dental milling machines, additive manufacturing machines, and the like for cloud computing, data exchange, remote control, and the like.

The extraoral x-ray system (1) has a control means for energizing the x-ray source (3), and linearly moving the x-ray ceph detector (8), and the x-ray ceph collimator (8).

The computerized extraoral x-ray system (1) shown in Fig. 2 is a multifunctional system for panoramic imaging, digital volume tomographic imaging, and **virtual** cephalometric imaging, computerized extraoral x-ray system (1) shown in Fig. 2 differs from the x-ray system (1) Fig. 1 in that it has no ceph unit attached on a cantilever arm (6).

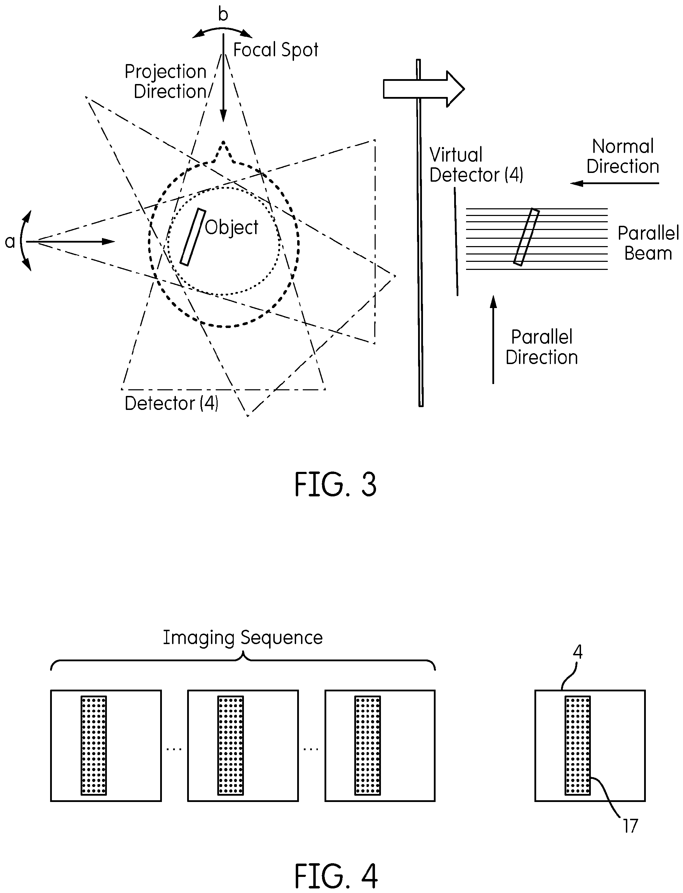

In the subsequent description, the computer-implemented method for generating a two-dimensional final image of at least a sub region of the maxillofacial region of a patient with x-ray cone beam CT, will be explained in more detail. The extraoral x-ray system (1) has a control means configured to execute this method. The method comprises the following steps:
In a defining step, a position and alignment of the 2D final image to be generated relative to said sub region of the dental maxillofacial region of the patient is defined as shown in the right side of Fig. 3. The 2D final image is essentially identical to an x-ray projection image of said sub region with parallel beam geometry.

In an acquisition step, a plurality of x-ray projection images are acquired by rotating the x-ray source (3) and the x-ray detector (4) around the head of the patient. The x-ray source (3) and the x-ray detector (4) are configured for the x-ray cone beam CT image acquisition as shown on the left side of Fig. 3.

During the acquisition step, the amounts of x-ray doses used for the acquisition of each of the x-ray projection images are varied as below:
A total amount of the x-ray dose is increased above a predetermined level for the x-ray projection images that are acquired when the projection direction is within a first predetermined range of angles (a) centered about a first direction which is parallel to the normal direction of the 2D final image to be generated. The normal direction is shown is shown in the right side of Fig. 3. The range of angles (a) centered about the first direction which is parallel to the normal direction can assume various values, preferably 45 degrees or less.

A total amount of the x-ray dose is reduced below the predetermined level for the x-ray projection images that are acquired when the projection direction is within a second predetermined range of angles (b) centered about a second direction which is perpendicular to the normal direction of the 2D final image to be generated. The range of angles (b) centered about a second direction which is perpendicular to the normal direction can assume various values, preferably 45 degrees or less.

Herein in the varying step, the speed of revolution and/or the emission of x-ray source (3) can be changed for adaptively varying the amounts of x-ray dose.

In an image reconstruction step the 2D final image on the virtual detector (4') plane and alignment as shown on the right side of Fig. 3 is generated as a **virtual projection** with parallel x-ray beam geometry of the sub region of the maxillofacial region of a patient by using the x-ray projection images acquired.

In the generating step, a volume image is reconstructed by using the plurality of x-ray projection images, and then the 2D final image is generated by virtually projecting with parallel x-ray beam geometry the sub region of the volume image.

In an acquisition step, the x-ray projection images are read out from the x-ray detector (4) (See Fig. 4) with a higher resolution compared to a predetermined resolution when the x-ray dose is relatively increased. And the x-ray projection images are read out from the x-ray detector (4) with a lower resolution compared to the said predetermined resolution when the x-ray dose is relatively decreased.

Flat panel sensor technologies of x-ray detector (4) for dental applications are based either on TFT (e.g. amorphous silicon or IGZO) or, depending on the size, on one or more CMOS wafers. Cesium iodide (CsI) is usually used as the scintillator material. They can be used in 1x1 binning for panorama imaging or e.g., 2x2 binning for DVT. The readout region (17) of the flat-panel detectors can be predefined (so-called partial mode). The larger the area to be read out, the lower the resulting frame rate. The dynamic range can be 16 bits. Multiple gain factors can be set and selected depending on the application.

According to the present invention the pixel binning in direction of movement of the gantry during gantry revolution around the patient as well as radiation dose can be changed.

According to the present invention the sensors spatial resolution can be changed during revolution of the x-ray detector and x-ray source around the patient. At the same time change adaption of x-ray dose per projection, such that SNR loss due to higher spatial resolution is at least partially compensated by a higher dose and vice versa. Spatial resolution is preferably changed by changing the binning of pixels on the sensor. Binning may be anisotropical. The resulting calculated volume may be anisotropical, e.g. the edge length of the voxels in the reconstructed volume differs in different spatial dimensions. In one preferred embodiment, the voxel size in one spatial dimension extends over the whole Field of View. In dental applications, this can be used to generate a cephalometric image of the dentomaxillofacial region of the patient.

According to the present invention the center position and width of the read-out region (14) can be changed during acquisition to enable orthoradial imaging of the object of interest.

The 2D final image can be a ceph image as shown 5. Alternatively, the 2D final image can be a smaller section of a ceph image showing at least the dental condition of the patient.

## Claims

1. A Computer-implemented method for generating a two dimensional final image (16) of at least a sub region of the maxillofacial region of a patient (P) with x-ray cone beam CT, the method comprising the steps of:
defining a position and alignment of the 2D final image to be generated relative to said sub region of the dental maxillofacial region of the patient, wherein the 2D final image essentially being identical to a x-ray projection image of said sub region with parallel beam geometry;
acquiring a plurality of x-ray projection images by rotating an x-ray source (3) and an x-ray detector (4) around the head of the patient, wherein the x-ray source and the x-ray detector are configured for a x-ray cone beam CT image acquisition;
varying, during the acquisition step, the amounts of x-ray doses used for the acquisition of each of the x-ray projection images such that
a total amount of the x-ray dose is increased above a predetermined level for the x-ray projection images that are acquired when the projection direction is within a first predetermined range of angles (a) centered about a first direction which is parallel to the normal direction of the 2D final image to be generated, and
a total amount of the x-ray dose is reduced below the predetermined level for the x-ray projection images that are acquired when the projection direction is within a second predetermined range of angles (b) centered about a second direction which is perpendicular to the normal direction of the 2D final image to be generated;
generating the 2D final image as a virtual projection with parallel x-ray beam geometry of the sub region of the maxillofacial region of a patient by using the x-ray projection images acquired.

2. The computer-implemented method according to claim 1, characterized that during the acquiring step
the x-ray projection images are read out from the x-ray detector with a higher resolution compared to a predetermined resolution when the x-ray dose is relatively increased; and
the x-ray projection images are read out from the x-ray detector with a lower resolution compared to the said predetermined resolution when the x-ray dose is relatively decreases.

3. The computer-implemented method according to any of the preceding claims, **characterized in that** in the generating step, a volume image is reconstructed by using the plurality of x-ray projection images, and then the 2D final image is generated by virtually projecting with parallel x-ray beam geometry the sub region of the volume image.

4. The computer-implemented method according to any of the preceding claims, **characterized in that** the 2D final image is a ceph image.

5. The computer-implemented method according to any of the preceding claims, **characterized in that** the varying step, the speed of revolution and/or the emission of x-ray sources is changed for adaptively varying the amounts of x-ray dose.

6. A computer program comprising computer-readable code which, when executed by a computer-assisted x-ray cone beam imaging system (1), causes the said system to perform the method of any one of the preceding claims.

7. An extraoral x-ray cone beam imaging system (1), comprising:
an x-ray source (3) and an x-ray detector (4) which are rotatably arranged around a patents' head;
a control means for controlling the x-ray source and the x-ray detector;
wherein the control means is configured to execute the method according to any one of the preceding claims.
